# FASCICULE DE BREVET EUROPEEN

(11) **EP 3 679 374 B1**
(45) Date de publication et mention de la délivrance du brevet: **23.06.2021**
(21) Numéro de dépôt: 18726776.0
(22) Date de dépôt: 19.05.2018
(51) Int. Cl.: G01N 1/40, G01N 33/574

(54) **DISPOSITIF NON-DESTRUCTIF D'ÉCHANTILLON POUR EXTRACTION DE MARQUEUR**
ZERSTÖRUNGSFREIE PROBENAHMEVORRICHTUNG ZUR EXTRAKTION EINES MARKERS
NON-DESTRUCTIVE SAMPLING DEVICE FOR EXTRACTION OF A MARKER

(30) Priorité: 06.09.2017 BE 201705633
(43) Date de publication de la demande: 15.07.2020
(73) Titulaire: Université de Liège, 4031 Angleur (BE)
(72) Inventeur: CASTRONOVO, Vincent, 400 Liege (BE); EMMERECHTS, Carl, 4500 Huy (BE)
(74) Mandataire: Peel, James Peter
(86) Numéro de dépôt international: PCT/EP2018/063244
(87) Numéro de publication internationale: WO 2019/048087

(56) Documents cités:
- GROSS: "Tissue fractionation by hydrostatic pressure cycling technology: the unified sample preparation technique for systems biology studies", JOURNAL OF BIOMOLECULAR TECHNIQUES, vol. 19, no. 3, 1 janvier 2008 (2008-01-01), page 189, XP055013562, ISSN: 1524-0215
- CAROL B. FOWLER ET AL: "Pressure-Assisted Protein Extraction: A Novel Method for Recovering Proteins from Archival Tissue for Proteomic Analysis", JOURNAL OF PROTEOME RESEARCH., vol. 11, no. 4, 7 mars 2012 (2012-03-07), pages 2602-2608, XP055458739, US ISSN: 1535-3893, DOI: 10.1021/pr201005t
- Brunella Costanza ET AL: "EXPEL: A Novel Non-Destructive Method for Mining Soluble Tumor Biomarkers", , 31 janvier 2015 (2015-01-31), XP055458607, Extrait de l'Internet: URL:chrome://ietab2/content/reloaded.html? url=http://easyposters.onetec.eu/posterPre sentation/ViewPoster.asp?evtid=%7B8dea35d1 -283e-40ee-a961-77293969f545%7D&UsrId=%7B5 379DF22-317F-453F-9512-A9CF13C6BE49%7D&Typ e=C&CatId=19&PosId=28 [extrait le 2018-03-12]
- B Costanza ET AL: "EXPEL: A Novel NonDestructive Method for Mining Soluble Tumor Biomarkers", , 28 février 2015 (2015-02-28), XP055458602, Extrait de l'Internet: URL:https://orbi.uliege.be/bitstream/2268/ 179914/1/O13.BWG2015.pdf [extrait le 2018-03-12]

## Description

Dispositif non-destructif d'échantillon pour extraction de marqueur.

La présente invention concerne une méthode, un dispositif et un appareillage non destructif d'échantillon, pour l'extraction de marqueurs à partir d'un échantillon. Plus particulièrement l'invention concerne une méthode, un dispositif et un appareillage pour l'extraction de biomarqueurs à partir d'échantillons biologiques et son utilisation pour le diagnostic à l'état précoce d'une maladie.

L'extraction de marqueurs en faible concentration dans un échantillon, quel que soit son origine se révèle problématique lorsque l'intégrité de l'échantillon doit être maintenu.

En particulier, La recherche de biomarqueurs ou marqueurs biologiques capables de détecter et d'évaluer le stade précoce d'une maladie tel que le cancer, ou prédire la résistance et la réponse au traitement reste un challenge.

Actuellement, la recherche non invasive de biomarqueurs se fait essentiellement au départ d'échantillons de sang, de sérum, d'urine ou de salive. Dans ces liquides, les biomarqueurs sont dilués jusqu'à un milliard de fois par rapport à la concentration au niveau du tissu malade ce qui rend leur détection à partir de ces milieux virtuellement impossible. Gross et al. (Journal of biomolecular techniques, 2008) décrit une méthode et un dispositif pour l'extraction de biomarqueurs d'un échantillon qui est pratiqué en soumettant l'échantillon à une variation de pression très haute à de cycles de 20 secondes.

La deuxième limitation est l'inaccessibilité d'échantillon de tissus tels que les biopsies solides, prioritairement destinés dans leur totalité à l'analyse pour du diagnostic. Ainsi, pour des raisons éthiques évidentes, ce matériel humain précieux n'est pas accessible pour la recherche de nouveaux biomarqueurs utiles et pertinents. Ceux-ci sont destinés dans leur intégrité pour l'évaluation histologique complète en test clinique. Ces échantillons sont d'ailleurs prélevés en quantité très faibles, de l'ordre de 1 à 125 mm³.

La détection de biomarqueurs est pourtant indispensable pour pouvoir poser un diagnostic individualisé et permettre le choix de la meilleure thérapie possible. Le but de la présente invention est de remédier à un problème de faible disponibilité de marqueurs lors de leur extraction à partir d'échantillons de quelque origine que ce soit, lorsque l'intégrité de l'échantillon doit être respectée.

Plus particulièrement, le but de la présente invention est de remédier au problème rencontré lors de l'utilisation de biopsie liquide telle que le sang, l'urine ou la salive avec une concentration trop faible en biomarqueurs, mais également de remédier à un manque de disponibilité de biopsie solide.

L'invention concerne dans un premier aspect une méthode aisée et rapide d'extraction de marqueur à partir d'échantillon comprenant les étapes suivantes:
- introduction d'un échantillon dans un panier-support jetable placé dans le logement d'une cellule d'un dispositif d'extraction muni d'une membrane flexible et
- immersion de l'échantillon dans un liquide préalablement introduit dans le logement de la cellule;
- fermeture hermétique de la cellule d'extraction après immersion de l'échantillon dans le liquide et application d'un cycle de pression sur la membrane flexible pendant une durée de 1 à 5 minutes, de préférence 3 minutes ;à une fréquence comprise entre 1 et 2 Hz, de préférence 1,5 Hz ;puis
- récupération totale ou partielle du liquide immergeant l'échantillon après application du cycle de pression sur la membrane et détection des marqueurs présents dans le liquide.
la pression hydrostatique appliquée sur la membrane du dispositif est comprise entre 2 et 5 bar, et est de préférence 2 bar.

Un cycle de pression signifie qu'une pression alternant entre maximum et minimum de pression est appliquée pendant un temps déterminé à une fréquence déterminée.

Par échantillon on entend tout type d'échantillon à l'état solide, issu d'aliments, de tissus végétal, de tissus biologiques, de composition minérale ou autre, susceptibles de comporter un ou plusieurs marqueurs en faible concentration dans l'échantillon. Plus particulièrement, l'échantillon de tissus biologique ou échantillon biologique est généralement issu d'un prélèvement chirurgical aussi dénommé biopsie, effectué par exemple au moyen d'une aiguille ou d'un bistouri. Le prélèvement de tissus peut avoir lieu au niveau de n'importe quel organe ou élément du corps humain ou animal comme par exemple du poumon, sein, foie, rein, utérus, prostate, moelle osseuse, muscle, colon, bronche et autre. L'échantillon de tissu peut être issu d'une tumeur cancéreuse.

Par liquide, on entend tout milieu de faible viscosité ou de viscosité proche de l'eau susceptible de solubiliser des marqueurs présents dans un échantillon solide immergé dans ce milieu. Par liquide on entend par exemple un milieu à base d'eau, d'alcool, d'huile et autres. Un exemple de milieu aqueux utilisé pour un échantillon biologique est un liquide physiologique.

Par liquide physiologique, on entend un liquide isotonique au sang ou présentant la même osmolarité que les principaux fluides corporels tels que le sang, l'urine, la salive. le liquide physiologique est par exemple composé d'eau distillée, de chlorure de sodium dilué à 0,9% de masse/volume de NaCl, soit 9 g/L. le liquide physiologique peut être plus complexe et comprendre par exemple du chlorure de potassium (KCI), chlorure de Calcium (Ca Cl₂), sulfate de Magnésium (MgSO₄) selon le tissu considéré.

Enfin le tampon phosphate salin (PBS) est également utilisé avec NaCl 9 g/L ou autre sels tels que KCl ou CaCl₂ en tant que liquide physiologique particulièrement avec des échantillons issus de tumeurs cancéreuses.

Le cycle de pression sur la membrane du dispositif d'extraction, doit permettre d'obtenir à partir du liquide, en particulier du liquide physiologique une quantité suffisante de marqueurs, en particulier des biomarqueurs, extrait de l'échantillon tout en évitant la dégradation de l'échantillon, en particulier l'échantillon de tissus ou biopsie. L'application d'une basse pression comprise entre 2 et 5 bar, sur la membrane flexible séparant le liquide permet une extrusion à partir de l'échantillon de marqueurs présents dans l'échantillon. Dans le cas d'un échantillon biologique, l'application d'une basse pression comprise entre 2 et 5 bar, sur la membrane flexible séparant le liquide permet une extrusion à partir de l'échantillon du tissu, d'un fluide interstitiel aux cellules du tissu dans lequel se trouvent les biomarqueurs d'intérêt. Le cycle de pression doit permettre néanmoins de maintenir la morphologie et l'antigènicité de l'échantillon de tissus aux fins d'analyses ultérieures.

Après avoir été soumise à une pression cyclique pendant une courte durée de 1 à 5 minutes, la pression est ramenée à pression atmosphérique et la cellule est déconnectée du cylindre à piston. Une fraction ou la totalité du liquide, en particulier le liquide physiologique est extrait de la cellule pour détection de marqueurs ou biomarqueurs, tandis que le panier muni de l'échantillon est retiré de la cellule pour permettre d'autres analyses ultérieures sur l'échantillon ayant maintenu son intégrité initiale. Dans le cas d'échantillon de tissus biologiques, ceux-ci seront soumis à des analyses cliniques ultérieures.

Par marqueur, on entend toute substance présente en faible concentration dans l'échantillon solide et susceptible de se solubiliser dans le liquide d'immersion de l'échantillon. Un marqueur peut être, par exemple, un composé biologique, toxique ou non présent dans un échantillon d'origine animale, végétale ou un aliment solide. Un marqueur peut également être un biomarqueur.

Par biomarqueur, on entend non seulement des protéines, mais également du DNA circulant, des exosomes, mi RNA, métabolites et molécules équivalentes se trouvant dans le liquide interstitiel aux cellules du tissu considéré.

La méthode selon l'invention présente l'avantage que l'échantillon est récupéré avec son intégrité morphologique et chimique ou biochimique après extraction et détection des marqueurs ou biomarqueurs.

La méthode d'extraction de marqueurs ou biomarqueurs a l'avantage d'être aisée et rapide. Elle ne prend que quelques minutes et n'occasionne aucun retard aux tests ultérieurs sur l'échantillon comme les tests cliniques dans le cas d'échantillons de tissus biologiques. Si une partie du liquide physiologique est maintenu dans la cellule d'extraction, il peut être stocké à basse température dans un liquide de cryopréservation comme l'azote liquide.

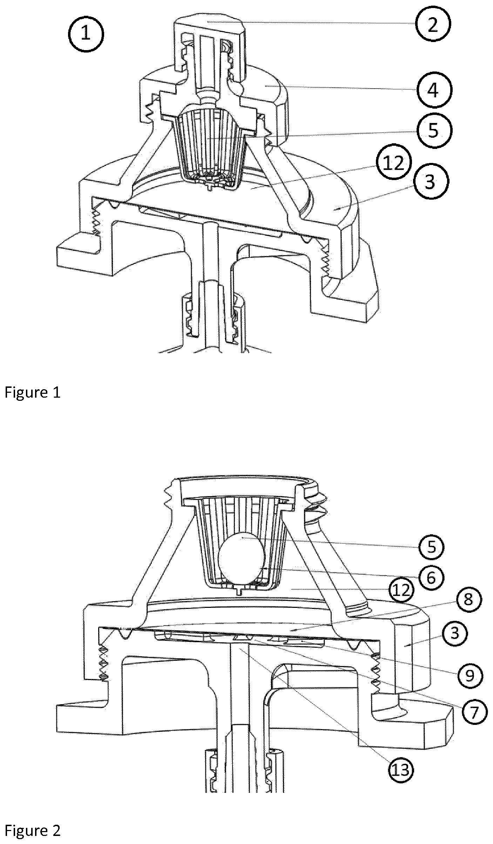

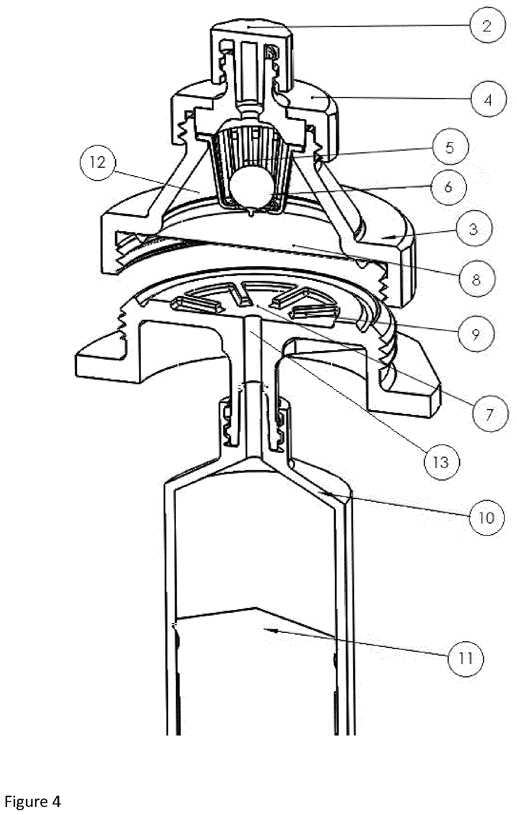

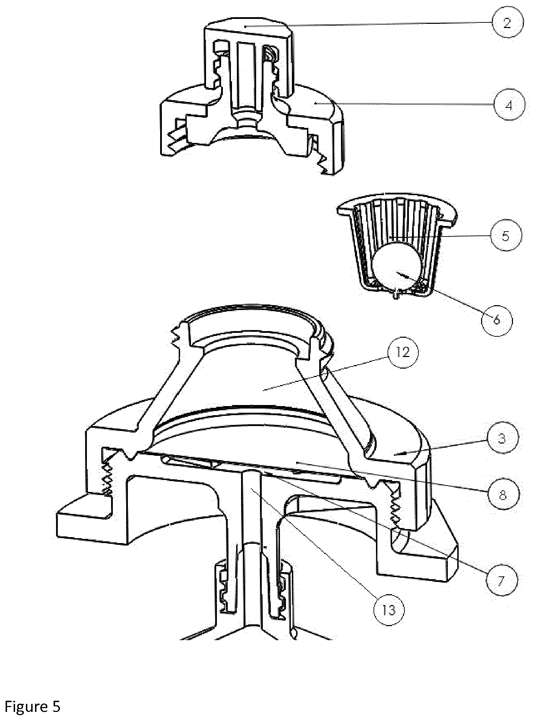

L'invention concerne dans un deuxième aspect, un dispositif pour l'extraction de marqueur qui pour l'intelligence de l'invention est illustré dans les figures 1 à 7.
Figure 1 : schéma de la cellule d'extraction selon l'invention
Figure 2 : étape d'introduction de l'échantillon dans la cellule d'extraction
Figure 3 : étape de fermeture hermétique de la cellule d'extraction au moyen d'un bouchon constitué de deux parties.
Figure 4 : illustration du système de pression sur le liquide dans lequel est plongé l'échantillon.
Figure 5 : étape de retrait après le cycle de pression, du panier-support comprenant l'échantillon.
Figure 6 : schéma de l'appareillage comprenant le dispositif d'extraction selon l'invention
Figure 7 : exemple de cellule d'extraction et d'appareillage selon l'invention.
Figure 8 et 9 comparaison analyse Immunohistochimie (IHC) sur échantillons de tissus biologiques ayant subi l'extraction de biomarqueurs selon invention et échantillons de référence non soumis à l'extraction.
Figure 10 : exemple d'utilisation du dispositif selon l'invention pour la détection de marqueurs protéiques.
Figure 11 : exemple d'utilisation du dispositif selon l'invention pour la détection de marqueurs métaboliques

Les figures servent à illustrer les modes de réalisation de l'invention, mais ne sont pas limitatives de l'invention.

Le dispositif d'extraction (1) comprend une cellule d'extraction (3) muni d'un logement (12) pour recevoir un panier amovible (5), ledit panier (5) étant configuré pour immerger un échantillon (6) dans un liquide préalablement introduit dans le logement (12) ;
Chaque élément appartenant au dispositif d'extraction est fabriqué par moulage à injection.

Dans le cas de dispositif à usage biologique, la fabrication est réalisée en salle blanche afin de répondre aux conditions de fabrication d'un dispositif médical. Une fois fabriqués, les éléments du dispositif d'extraction sont lavés au moyen d'un solvant organique tel que de l'isopropanol dans un bain ultrasonique. Le niveau de stérilisation des éléments ainsi fabriqué doit être conforme au standard DIN EN ISO 17665 pour dispositifs médicaux et être tel qu'aucune trace d'ADN ne soit identifiable.

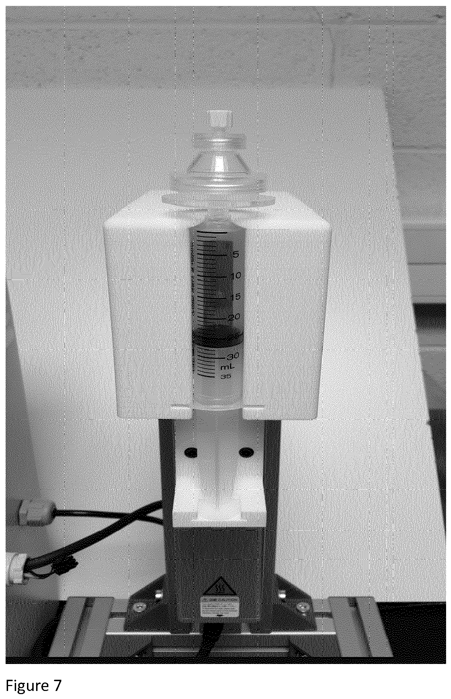

La cellule d'extraction est de préférence fabriquée en un matériau polymérique transparent tel que le polyméthacrylate comme illustrée à la figure 7.

Le dispositif d'extraction (1) comprend également une cavité (7) séparée du logement (12) de la cellule d'extraction au moyen d'une membrane flexible (8).

L'ensemble constitué de la cavité et de la membrane flexible permet une compression ou une dilatation du liquide et de l'échantillon de tissus présent dans la cellule d'extraction hermétiquement fermée.

Le volume de la cavité est compris entre 3 et 10mL, de préférence 4.5mL.

La membrane (8) est en matériau polymérique flexible et résistant à une déformation de 50% à 200% de sa surface. La membrane doit résister à la déformation lors de la compression du liquide ou de la dilatation du liquide lors d'un stockage de la cellule d'extraction (3) dans l'azote liquide par exemple. De préférence, la flexibilité de la membrane permet un taux de déformation de 1 à 2 fois sa surface.

La membrane est de préférence en silicone avec une épaisseur qui peut varier de 50 à 1000 micron avec une préférence de 100 microns.

La membrane flexible, de préférence en silicone, est fixée entre deux pièces d'assemblement et est agencée par exemple à la base de la cellule d'extraction (3), au moyen d'une rainure en V. Les deux pièces d'assemblement peuvent être optionnellement désolidarisées.

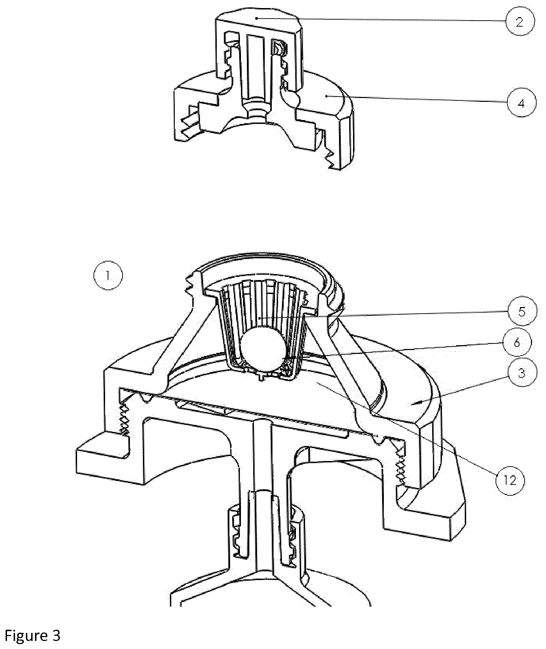

Le dispositif d'extraction (1) comme illustré à la figure 3, comprend également un bouchon étanche, agencé sur la cellule d'extraction (3) pour fixer le panier-support (5) amovible dans le logement(12) et maintenir le liquide à l'intérieur du logement (12) durant le cycle de pression.

Selon un mode préféré, le bouchon étanche comprend deux parties, une partie supérieure (2) ou bouchon Luer Lock agencé sur une partie inférieure (4) configurée pour permettre l'extraction du liquide de la cellule et pour maintenir l'étanchéité de la cellule d'extraction durant le cycle de pression.

La partie inférieure du bouchon est de préférence vissée sur la cellule d'extraction au moyen d'un système cône à cône permettant ainsi l'étanchéité de la cellule d'extraction.

Le panier-support (5) est fabriqué par moulage à injection, en matière plastique également, de préférence du polypropylène. Dans un mode de réalisation préféré le panier-support est jetable.

De préférence, le panier est muni de nervures verticales permettant l'immersion de l'échantillon de tissus dans le liquide physiologique. Les nervures du récipient doivent avoir une largeur adaptée à la taille de l'échantillon pour permettre à l'échantillon de rester dans le panier tout en étant immergé dans le liquide physiologique.

Le volume du panier-support (5) dans le logement (12) de la cellule d'extraction (3) représente entre 10 et 50%, de préférence 20% du volume du logement à l'intérieur de la cellule d'extraction. Le volume du panier est également lié à la taille de l'échantillon. Selon un autre mode de réalisation préféré, le dispositif d'extraction comprend un cylindre (10) muni d'un piston (11), ledit cylindre étant connecté à la cavité (9) à l'orifice (13). L'ensemble cavité-membrane permet d'obtenir une répartition uniforme de la pression sur le liquide et une meilleure performance d'extraction des marqueurs De préférence, le volume de la cavité et du cylindre au-dessus du piston est rempli d'air, mais tout autre gaz inerte peut également être utilisé tel que azote, argon ou équivalent. Le volume total de la cavité et du cylindre rempli d'air peut varier de 20 à 100 ml, mais est de préférence de l'ordre de 30 ml.

Dans un mode encore plus préféré du dispositif de l'invention, le cylindre muni du piston est une seringue médicale jetable.

Le cylindre est généralement connecté verticalement à la cellule d'extrusion, mais tout autre configuration, comme par exemple une configuration horizontale est également possible.

Selon un autre mode de réalisation préféré la cavité comprend un ou plusieurs raidisseurs (9). Lorsque les raidisseurs sont un multiple de 2, ils sont de préférence disposés en étoile autour de l'orifice (13) de connexion du cylindre (10). L'utilisation de raidisseur permet d'éviter avantageusement un collement de la membrane à la paroi de la cavité.

Dans le cas de dispositif à usage biologique, le liquide sera de préférence un liquide physiologique pour l'extraction de biomarqueurs à partir d'un échantillon de tissus biologiques immergé dans ce liquide physiologique.

La présente invention concerne également dans un 3^{e} aspect, un dispositif d'extraction de biomarqueurs pour diagnostic précoce de maladies à un stade primaire ou pour traitement de maladie, plus particulièrement pour le traitement de cancer.

La présente invention concerne également dans un 4^{e} aspect, une utilisation du dispositif pour la détection de biomarqueurs protéiques, métabolique moléculaires à partir d'échantillon de tissus ou biopsie solide.

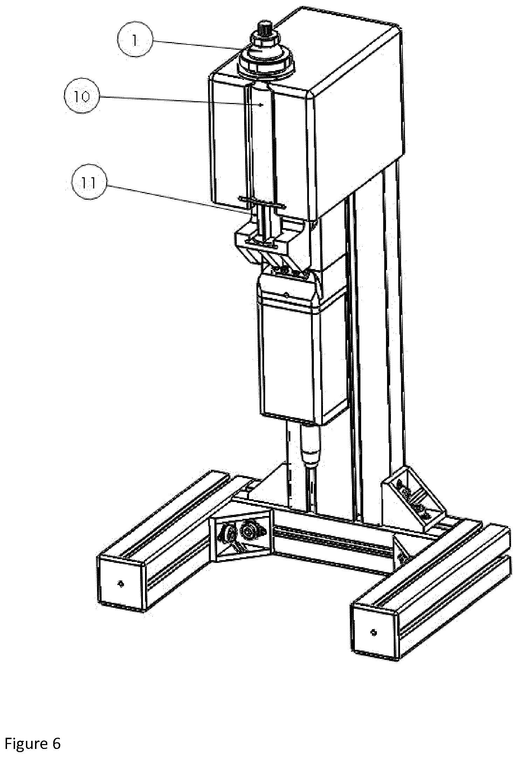

Enfin la présente invention concerne un 5^{e} aspect, un appareillage comme illustré à la figure 6 intégrant le dispositif d'extraction ainsi que des moyens pour actionner le piston de pression comme par exemple un verrin. L'appareillage peut comprendre également un module automatique de contrôle de paramètres.

La figure 7 représente un exemple d'un appareillage comprenant la cellule d'extraction ainsi qu'une seringue chirurgicale de 30mL comme cylindre à piston.

### Exemples

La méthode, le dispositif et l'appareillage ont été testés sur une grande série d'échantillons de tissus biologiques obtenus à partir de tumeurs colorectales primaires (CRC) et de métastase de foie (CRC-LM). Le liquide extrudé à partir des échantillons est un matériel unique pour la détection de biomarqueurs et n'interfère nullement dans la morphologie des tissus pour les tests cliniques ultérieurs comme illustré ci-après.

### 1.Application de la méthode d'extraction selon l'invention.

Des biopsies fraichement prélevées chirurgicalement ont été collectées immédiatement après opération, découpées en échantillon de 3 mm³ et placé dans une cellule de 4,5 mL. Quatre mL de tampon PBS hypertonic avec NaCl, 4.5 g dans 500 mL (Weestburg) a été ajouté dans la cellule en même temps que l'échantillon dans le panier. Indépendamment, le piston de la seringue est alors déplacé jusqu'au marquage de 15mL. La seringue est ensuite connectée à la cellule d'extraction. L'échantillon est ensuite soumis à une pression alternative à une fréquence de 1 Hz pendant une minute par le déplacement du piston dans la seringue entre le marquage 15mL et 7,5 mL. Lors de mesures effectuées séparément avec un manomètre connecté, il apparait que ce changement de volume correspond à une variation de pression effective entre 2 bar (état de compression) et 1 bar bar (état relaxé) et 1 bar au point initial lorsque le piston se trouve à la marque 15mL. La procédure est répétée 3 fois pour chaque exemple. Les fluides d'extraction ont ensuite été conservés à -20°C. Toute la procédure a duré 3 minutes et l'échantillon est restitué pour évaluation clinique. Un protocole semblable a été appliqué à des échantillons issus de tumeurs du cancer du sein humain, du cancer colorectal primaire et des métastases de foie et du colon ainsi que sur leur contreparties normales adjacentes.

### 2. Comparaison des résultats d'Immunohistochimie (IHC) sur échantillons ayant/n'ayant pas subit la méthode d'extraction selon l'invention.

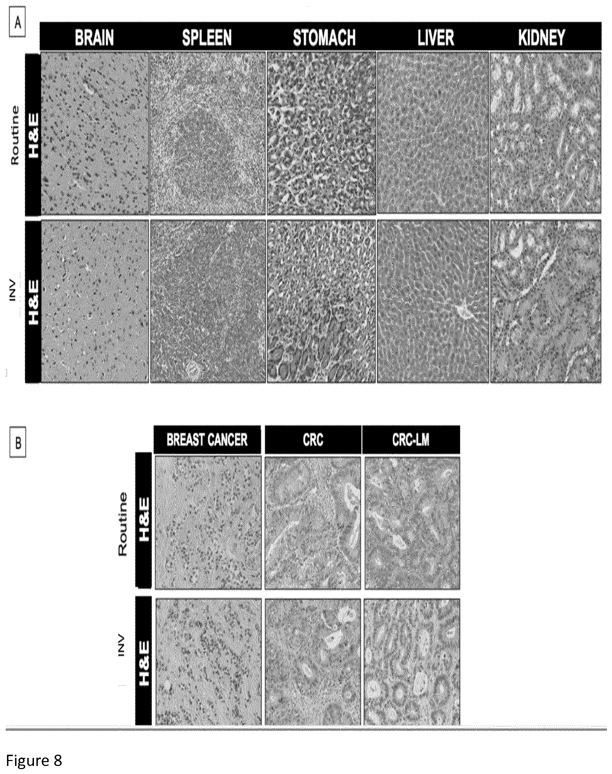

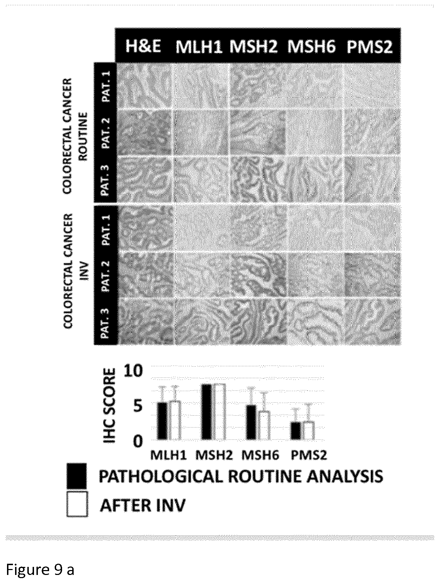

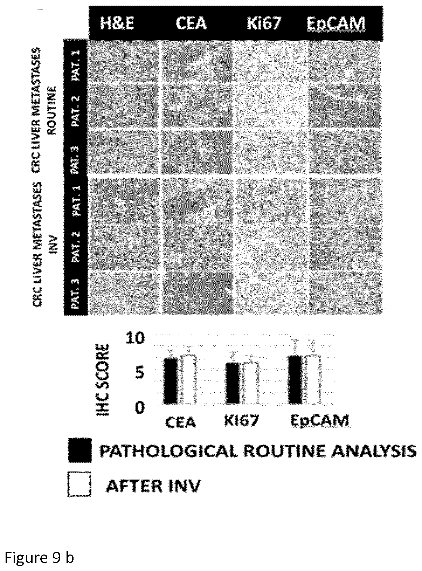

Pour vérifier que la méthode selon l'invention ne change pas la morphologie et antigénicité des échantillons tissulaires, une analyse comparative de différents tissus humain et de souris ont été soumis à la méthode. Chaque échantillon de tissus a été divisé en deux; d'une part pour analyse pathologique de routine et d'autre part pour la méthode d'extraction selon l'invention aussi dénommée INV dans les figures 8 et 9. Les échantillons pour l'analyse de routine et pour la méthode d'extraction selon l'invention sont soumis à la même Immunohistochimie (IHC) selon le protocole connu de l'homme du métier et décrit par A.Bellahcene et V.Castronovo dans Am. J Pathol 1955 jan ; 146(1) : 95-100 .

Dans la figure 8, des échantillons de tumeurs colorectales primaires (n=10, panneau gauche) et des échantillons de foie métastasés (n=10, panneau droit) ont été soumis à l'analyse clinique de routine et à la méthode d'extraction selon l'invention suivi de l'analyse hematoxylin/eosin (H&E) et immunolabelling pour les marqueurs identifiés (MLH1, MSH2, MSH6, PMS2). L'évaluation quantitative pour chaque marqueur a été effectuée selon les méthodes connues de l'homme du métier et décrit par D.Waltregny & cie dans J Natl Cancer Inst. 1998 Jul 1;90(13):1000-8.

Des échantillons de 3 patients ont été collectés dans chaque cas. Chaque image IHC a été évaluée pour son intensité en utilisant l'échelle suivante (0 pour aucune trace, 1 pour faible, 2 pour modéré et 3 pour fort). Les tissus ont été également évalué pour l'étendue de leur positivité en utilisant l'échelle 1= 0-33%, 2= 33-66%, 3=66-100%. Les valeurs obtenues par chacune des deux évaluations sont multipliées pour donner le HIC score reproduit à la figure 8.

Dans la figure 9, des échantillons de tissus de souris présentant un cancer du sein (n=3), un cancer colorectal (CRC ;n=3), un cancer du foie en métastase (CRC-LM ; n=3) ont été l'objet soit au test de routine soit à la méthode d'extraction suivi de l'analyse hematoxylin/eosin (H&E).

Comme illustré dans les figures 8 et 9 aucune différence significative n'a été mise en évidence concernant la structure du tissu (H&E) et l'intensité des marqueurs choisis.

### 3.utilisation du dispositif pour l'Identification de marqueurs protéiques et métaboliques au niveau du cancer du sein humain.

Dix prélèvements de cancer du sein et leur tissus non tumoral voisin ont été traités selon le protocole décrit à l'exemple 1 puis soumis au dispositif et méthode selon l'invention. Le liquide obtenu après application du dispositif d'extraction a alors été analysé par spectrométrie de masse pour la détection de protéines solubles et par Résonnance Magnétique Nucléaire pour la détection de métabolites. Les marqueurs identifiés à partir du liquide obtenu des tissus cancéreux ont alors été comparé à ceux des tissus normaux.

### 3.1 détection des marqueurs protéiques

Parmi les protéines détectées dans les tissus cancéreux, on identifie par exemple les Periostin, comp, Ezrin, Raxidin, Versican, Tenascin. Ces protéines sont généralement présentes dans l'espace extracellulaire des tissus comme illustrés dans le tableau 1 ci-dessous reprenant des extraits de Pubmed :

**Tableau 1 : protéines identifiées dans les tissus du cancer du sein**

| ***Periostin*** 49 Results on Pubmed | | ***COMP .*** 4 results on Pubmed |
|---|---|---|
| • Protein involved in osteoblast adhésion. | | • Localization: extracellular space |
| • Major component of ECM. | | • Tumor development |
| • Secreted by fibroblasts | | • Metastases dissémination |
| • Generally associated with poor patient outcome. | | |

| ***Ezrin*** 114 results on Pubmed | | ***Radixin*** 63 results on Pubmed |
|---|---|---|
| • Localization: plasma membrane Major association: | | • Localization: cell membrane, extracellular space |
| | | • **Ezrin-radixin-moesin (ERM)** plys important role in the maintenance of cytoskeleton structure and cells movement. It also involved in breast cancer invasion and metastasis and may be a potential biomarker. This axis is regulated by miR-200c |
| • EMT | | |
| • Cell motility | | |
| • Disease progression (ovarian cancer) | | |
| • migration & invasion | | |
| | | |

| ***Versicon*** 36 results on Pubmed | | ***Tenascin*** 158 results on pumed |
|---|---|---|
| • Localization: extracellular matrix Major association: | | Localization: extracellular space *Function:* |
| • Cell motility | | Implicated in guidance of migrating neurons as well as axons during development Function in tumors: Stimulates angiogenesis |
| • Cell differentiation • Cell growth | | |
| • TGFB-1 induced | | |

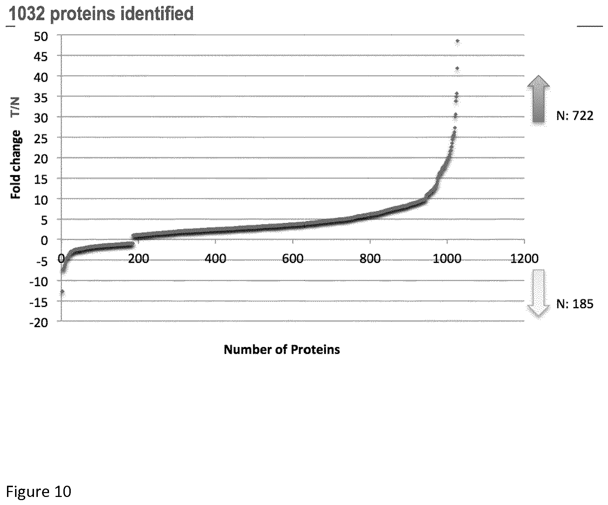

La figure 10 illustre le nombre de marqueurs protéiques identifiés dans le liquide obtenu suite au dispositif et à la méthode d'extraction selon l'invention. Les marqueurs identifiés à partir du liquide dans lequel un tissu cancéreux a été plongé, sont comparés à ceux extraits des tissus normaux.

1032 protéines ont ainsi été identifiées à partir des tissus cancéreux et sont exprimées de manière différentielle (722 augmentées et 185 diminuées) dans la figure 10

### 3.2 détection des marqueurs métaboliques

L'Ingenuity Patway Analysis a été utilisé pour l'interprétatin des données récoltées et leur analyse au moyen du logiciel IPA Core Analysis

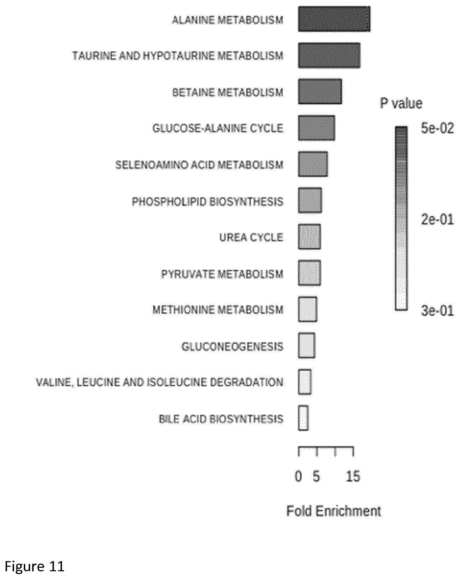

Dans la figure 11 sont illustrés les marqueurs métaboliques identifiés selon la méthode et le dispositif d'extraction selon l'invention. On assiste à une altération et une augmentation des métabolites dans le cas du cancer mammaire. L'alanine, la taurine et l'hypotaurine, la betaine, le glucose alanine sont les marqueurs ayant subi le plus important up-regulation comme le reflète la valeur de P.

La valeur de P indique quelle molécule est associée significativement avec des moléculesde référence introduites et cela relativement à toutes les molécules fonctionnelles caractérisées. Le fold enrichment indique la signification statistique de la présence du métabolite dans l'échantillon.

## Revendications

1. Méthode d'extraction de marqueur comprenant :
- l'introduction d'un échantillon dans un panier-support amovible placé dans le logement d'une cellule d'un dispositif d'extraction muni d'une membrane flexible et
- immersion de l'échantillon dans un liquide préalablement introduit dans le logement;
- fermeture hermétique de la cellule d'extraction après immersion de l'échantillon dans le liquide et
- application d'un cycle de pression sur la membrane flexible pendant une durée de 1 à 5 minutes, à une fréquence comprise entre 1 et 2 Hz ; puis
- récupération totale ou partielle du liquide immergeant l'échantillon après le cycle de pression et détection des marqueurs présents dans le liquide physiologique ;
**caractérisé en ce que** :
la pression appliquée sur la membrane est comprise entre 2 et 5 bar.

2. Méthode selon l'une quelconque des revendications précédentes **caractérisée en ce que** la durée du cycle de pression est de 3 minutes.

3. Méthode selon l'une quelconque des revendications précédentes **caractérisée en ce que** l'échantillon est un échantillon de tissu biologique et le liquide, un liquide physiologique pour l'extraction de biomarqueurs.

4. Méthode selon la revendication 3 **caractérisée en ce que** l'échantillon de tissu biologique est extrait d'une tumeur cancéreuse.

5. Méthode selon l'une quelconque des revendications 3 et 4 **caractérisée en ce que** le liquide physiologique est un tampon PBS avec NaCI 9 g/L.

6. Dispositif (1) d'extraction de marqueur comprenant :
- une cellule d'extraction (3) muni d'un logement (12) pour recevoir un panier-support amovible (5), ledit panier (5) étant configuré pour immerger un échantillon (6) de dans un liquide préalablement introduit dans le logement (12) ;
- une cavité (7) séparée du logement de la cellule d'extraction (3) au moyen d'une membrane flexible (8), permettant une compression ou une dilatation du liquide ; et
- un bouchon étanche (2) agencé sur la cellule d'extraction (3) pour fixer le panier - support amovible (5) dans le logement (12).

7. Dispositif selon la revendication 6 **caractérisé en ce que** le bouchon étanche comprend deux parties, une partie supérieure ou bouchon Luer Lock (2) agencé sur une partie inférieure (4) configurée pour maintenir l'étanchéité de la cellule d'extraction durant le cycle de pression et pour permettre l'extraction du liquide en fin de cycle.

8. Dispositif selon l'une quelconque des revendications 6 à 7 **caractérisé en ce qu'**il comprend également un cylindre (10) à piston (11) connecté à la cavité (7) à l'orifice (13).

9. Dispositif selon la revendication 8 **caractérisé en ce que** la cavité (7) et le volume du cylindre (10) au-dessus du piston sont rempli d'air.

10. Dispositif selon l'une quelconque des revendications 6 à 9 **caractérisé en ce que** la cavité (7) comprend également au moins un raidisseur (9).

11. Dispositif selon la revendication 10 **caractérisé en ce que** au moins deux raidisseurs sont disposés en étoile autour de l'orifice (13) de connexion du cylindre (10)

12. Dispositif selon l'une quelconque des revendications précédentes 6 à 11 **caractérisé en ce que** le panier-support (5) est muni de nervures verticales permettant l'immersion de l'échantillon dans le liquide.

13. Dispositif selon la revendication précédente 12 **caractérisé en ce que** les nervures du panier-support (5) doivent avoir une largueur adaptée à la taille de l'échantillon.

14. Dispositif selon l'une quelconque des revendications précédentes 6 à 13 **caractérisé en ce que** le panier-support (5) est en matière plastique, de préférence du polypropylène.

15. Dispositif selon l'une quelconque des revendications précédentes 6 à 14 **caractérisé en ce que** la flexibilité de la membrane (8) permet un taux de déformation de 1 à 2 fois sa surface.

16. Dispositif selon l'une quelconque des revendications précédentes 8 à 15 dans lequel le cylindre (10) est connecté verticalement à la cellule d'extrusion (3).

17. Dispositif selon l'une quelconque des revendications précédentes 8 à 16 dans lequel le cylindre (10) muni du piston (11) est une seringue jetable.

18. Dispositif d'extraction selon l'une quelconque des revendications précédentes 6 à 17 **caractérisé en ce que** l'échantillon est un échantillon de tissu biologique et le liquide, un liquide physiologique pour l'extraction de biomarqueurs.

19. Dispositif d'extraction de biomarqueur selon la revendication 18 pour le diagnostic précoce de cancer au stade primaire.

20. Dispositif d'extraction de biomarqueur selon la revendication 18 pour le traitement du cancer.

21. Utilisation du dispositif selon la revendication 18 pour la détection de biomarqueurs protéiques, métabolique moléculaires à partir de biopsie solide.

22. Appareillage comprenant le dispositif d'extraction de marqueurs selon l'une quelconque des revendications 8 à 18 ainsi qu'un moyen pour actionner la course du piston.

23. Appareillage selon la revendication 22 **caractérisé en ce que** le moyen pour actionner la course du piston est un verrin.

## Patentansprüche

1. Verfahren zur Extraktion von Markern, umfassend:
- Einbringen einer Probe in einen herausnehmbaren Trägerkorb, der im Aufnahmeraum einer Zelle einer Extraktionsvorrichtung angeordnet ist, wobei letztere mit einer nachgiebigen Membran versehen ist, und
- Eintauchen der Probe in eine Flüssigkeit, welche zuvor in den Aufnahmeraum eingebracht wurde,
- hermetisches Verschließen der Extraktionszelle, nachdem die Probe in die Flüssigkeit eingetaucht wurde, und
- Anwenden eines Druckzyklus auf die nachgiebige Membran während einer Dauer von 1 bis 5 Minuten, mit einer Frequenz im Bereich von 1 bis 2 Hz; und danach
- vollständiges oder teilweises Gewinnen der Flüssigkeit, in welche die Probe eingetaucht wurde, im Anschluss an den Druckzyklus sowie Nachweisen der Marker, welche in der physiologischen Flüssigkeit enthalten sind;
**dadurch gekennzeichnet, dass**:
der Druck, welcher auf die Membran angewendet wird, im Bereich von 2 bis 5 bar liegt.

2. Verfahren nach Anspruch 1, **dadurch gekennzeichnet, dass** die Dauer des Druckzyklus 3 Minuten beträgt.

3. Verfahren nach einem beliebigen der vorhergehenden Ansprüche, **dadurch gekennzeichnet, dass** es sich bei der Probe um eine biologische Gewebeprobe und bei der Flüssigkeit um eine physiologische Flüssigkeit zur Extraktion von Biomarkern handelt.

4. Verfahren nach Anspruch 3, **dadurch gekennzeichnet, dass** die biologische Gewebeprobe aus einem bösartigen Tumor entnommen wird.

5. Verfahren nach einem beliebigen der Ansprüche 3 und 4, **dadurch gekennzeichnet, dass** es sich bei der physiologischen Flüssigkeit um einen PBS-Puffer mit NaCl 9 g/L handelt.

6. Vorrichtung (1) zur Extraktion von Markern, umfassend:
- eine Extraktionszelle (3), die mit einem Aufnahmeraum (12) versehen ist, um einen herausnehmbaren Trägerkorb (5) aufzunehmen, wobei der Korb (5) dafür ausgelegt ist, eine Probe (6) in eine Flüssigkeit einzutauchen, welche zuvor in den Aufnahmeraum (12) eingebracht wurde;
- einen Hohlraum (7), welcher von dem Aufnahmeraum der Extraktionszelle (3) mittels einer nachgiebigen Membran (8) getrennt ist, die eine Kompression oder eine Ausdehnung der Flüssigkeit ermöglicht; und
- eine abdichtende Verschlusskappe (2), die auf der Extraktionszelle (3) angeordnet ist, um den herausnehmbaren Trägerkorb (5) in dem Aufnahmeraum (12) zu festzuhalten.

7. Vorrichtung nach Anspruch 6, **dadurch gekennzeichnet, dass** die abdichtende Verschlusskappe zwei Abschnitte umfasst, einen oberen Abschnitt oder Luer-Lock-Verschlusskappe (2), der auf einem unteren Abschnitt (4) angeordnet ist, welcher dafür ausgelegt ist, die Dichtigkeit der Extraktionszelle während des Druckzyklus sicherzustellen und eine Entnahme der Flüssigkeit am Ende des Zyklus zu ermöglichen.

8. Vorrichtung nach einem beliebigen der Ansprüche 6 bis 7, **dadurch gekennzeichnet, dass** sie weiterhin einen Zylinder (10) mit Kolben (11) umfasst, der im Bereich der Öffnung (13) mit dem Hohlraum (7) verbunden ist.

9. Vorrichtung nach Anspruch 8, **dadurch gekennzeichnet, dass** der Hohlraum (7) und das Volumen des Zylinders (10) oberhalb des Kolbens mit Luft gefüllt sind.

10. Vorrichtung nach einem beliebigen der Ansprüche 6 bis 9, **dadurch gekennzeichnet, dass** der Hohlraum (7) weiterhin mindestens ein Versteifungsorgan (9) umfasst.

11. Vorrichtung nach Anspruch 10, **dadurch gekennzeichnet, dass** mindestens zwei Versteifungsorgane sternförmig um die Verbindungsöffnung (13) des Zylinders (10) angeordnet sind.

12. Vorrichtung nach einem beliebigen der vorhergehenden Ansprüche 6 bis 11, **dadurch gekennzeichnet, dass** der Trägerkorb (5) mit vertikalen Rippen versehen ist, welche das Eintauchen der Probe in die Flüssigkeit ermöglichen.

13. Vorrichtung nach dem vorhergehenden Anspruch 12, **dadurch gekennzeichnet, dass** die Rippen des Trägerkorbs (5) eine Breite haben müssen, welche an die Größe der Probe angepasst ist.

14. Vorrichtung nach einem beliebigen der vorhergehenden Ansprüche 6 bis 13, **dadurch gekennzeichnet, dass** der Trägerkorb (5) aus Kunststoff ist, vorzugsweise aus Polypropylen.

15. Vorrichtung nach einem beliebigen der vorhergehenden Ansprüche 6 bis 14, **dadurch gekennzeichnet, dass** die Nachgiebigkeit der Membran (8) einen Verformungsgrad ermöglicht, welcher dem 1- bis 2-fachen ihrer Oberfläche entspricht.

16. Vorrichtung nach einem beliebigen der vorhergehenden Ansprüche 8 bis 15, wobei der Zylinder (10) in vertikaler Richtung mit der Extrusionszelle (3) verbunden ist.

17. Vorrichtung nach einem beliebigen der vorhergehenden Ansprüche 8 bis 16, wobei es sich bei dem Zylinder (10), welcher mit dem Kolben (11) versehen ist, um eine Einwegspritze handelt.

18. Extraktionsvorrichtung nach einem beliebigen der vorhergehenden Ansprüche 6 bis 17, **dadurch gekennzeichnet, dass** es sich bei der Probe um eine biologische Gewebeprobe und bei der Flüssigkeit um eine physiologische Flüssigkeit zur Extraktion von Biomarkern handelt.

19. Vorrichtung zur Extraktion von Biomarkern nach Anspruch 18, zur Früherkennung von Krebserkrankungen im Primärstadium.

20. Vorrichtung zur Extraktion von Biomarkern nach Anspruch 18, zur Behandlung der Krebserkrankung.

21. Verwendung der Vorrichtung nach Anspruch 18 zum Nachweis molekularer proteinartiger Stoffwechselbiomarker ausgehend von einer festen Gewebeprobe.

22. Anlage, welche die Vorrichtung zur Extraktion von Markern nach einem beliebigen der Ansprüche 8 bis 18 sowie ein Mittel zur Durchführung des Kolbenhubs umfasst.

23. Anlage nach Anspruch 22, die **dadurch gekennzeichnet ist, dass** es sich bei dem Mittel zur Durchführung des Kolbenhubs um einen Stellzylinder handelt.

## Claims

1. Method for extracting a marker, comprising:
- introducing a sample into a removable support basket placed in the housing of a cell of an extraction device provided with a flexible membrane and
- immersing the sample in a liquid previously introduced into the housing;
- hermetically closing the extraction cell after immersion of the sample in the liquid and
- applying a pressure cycle on the flexible membrane for a period of 1 to 5 minutes, at a frequency of between 1 and 2 Hz; then
- totally or partially recovering the liquid immersing the sample after the pressure cycle, and detecting the markers present in the physiological liquid;
**characterized in that**:
the pressure applied on the membrane is between 2 and 5 bar.

2. Method according to Claim 1, **characterized in that** the duration of the pressure cycle is 3 minutes.

3. Method according to either one of the preceding claims, **characterized in that** the sample is a biological tissue sample and the liquid is a physiological liquid for the extraction of biomarkers.

4. Method according to Claim 3, **characterized in that** the biological tissue sample is extracted from a cancerous tumor.

5. Method according to either one of Claims 3 and 4, **characterized in that** the physiological liquid is a PBS buffer with 9 g/l NaCl.

6. Device (1) for extracting a marker, comprising:
- an extraction cell (3) provided with a housing (12) for receiving a removable support basket (5), said basket (5) being configured to immerse a sample (6) in a liquid previously introduced into the housing (12);
- a cavity (7) separated from the housing of the extraction cell (3) by means of a flexible membrane (8), allowing a compression or a dilation of the liquid; and
- a leaktight cap (2) placed on the extraction cell (3) for attaching the removable support basket (5) in the housing (12).

7. Device according to Claim 6, **characterized in that** the leaktight cap comprises two portions, an upper portion or Luer Lock cap (2) placed on a lower part (4) configured to maintain the leaktightness of the extraction cell during the pressure cycle and to allow the extraction of the liquid at the end of the cycle.

8. Device according to either one of Claims 6 and 7, **characterized in that** it also comprises a piston (11) cylinder (10) connected to the cavity (7) at the orifice (13) .

9. Device according to Claim 8, **characterized in that** the cavity (7) and the volume of the cylinder (10) above the piston are filled with air.

10. Device according to any one of Claims 6 to 9, **characterized in that** the cavity (7) also comprises at least one stiffener (9).

11. Device according to Claim 10, **characterized in that** at least two stiffeners are placed in a star pattern around the cylinder (10) connection orifice (13).

12. Device according to any one of Claims 6 to 11, **characterized in that** the support basket (5) is provided with vertical ribs allowing the immersion of the sample in the liquid.

13. Device according to the preceding Claim 12, **characterized in that** the ribs of the support basket (5) must have a width suitable for the size of the sample.

14. Device according to any one of Claims 6 to 13, **characterized in that** the support basket (5) is made of plastic, preferably polypropylene.

15. Device according to any one of Claims 6 to 14, **characterized in that** the flexibility of the membrane (8) allows a degree of deformation of 1 to 2 times its surface area.

16. Device according to any one of the preceding Claims 8 to 15, wherein the cylinder (10) is vertically connected to the extrusion cell (3).

17. Device according to any one of the preceding Claims 8 to 16, wherein the cylinder (10) provided with the piston (11) is a disposable syringe.

18. Extraction device according to any one of the preceding Claims 6 to 17, **characterized in that** the sample is a biological tissue sample and the liquid is a physiological liquid for the extraction of biomarkers.

19. Device for extracting a biomarker according to Claim 18, for the early diagnosis of cancer at the primary stage.

20. Device for extracting a biomarker according to Claim 18, for cancer treatment.

21. Use of the device according to Claim 18, for detecting molecular metabolic and protein biomarkers from a solid biopsy.

22. Apparatus comprising the device for extracting markers according to any one of Claims 8 to 18, and also a means for actuating the travel of the piston.

23. Apparatus according to Claim 22, **characterized in that** the means for actuating the travel of the piston is a jack.
